Europäisches Patentamt

European Patent Office

Office européen des brevets

(19)

(11) **EP 1 552 803 A1**

(12) **EUROPEAN PATENT APPLICATION**
published in accordance with Art. 158(3) EPC

(43) Date of publication:
**13.07.2005 Bulletin 2005/28**

(51) Int Cl.⁷: **A61F 13/514**

(21) Application number: **03795283.5**

(86) International application number:
**PCT/JP2003/011242**

(22) Date of filing: **03.09.2003**

(87) International publication number:
**WO 2004/024048 (25.03.2004 Gazette 2004/13)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **13.09.2002 JP 2002269276**

(71) Applicant: **UNI-CHARM CORPORATION**
**Kawanoe-shi Ehime 799-0111 (JP)**

(72) Inventors:
• **SUZUKI, Sachiyo,**
**Technical Center, Uni-Charm Corp.**
**Mitoyo-gun, Kagawa 769-1602 (JP)**
• **ISHIKAWA, Hiroki,**
**Technical Center, Uni-Charm Corp**
**Mitoyo-gun, Kagawa 769-1602 (JP)**
• **SAKAGUCHI, Satoru,**
**Technical Ctr., Uni-Charm Corp.**
**Mitoyo-gun, Kagawa 769-1602 (JP)**
• **YUASA, Kaori,**
**Technical Center, Uni-Charm Corp.**
**Mitoyo-gun, Kagawa 769-1602 (JP)**

(74) Representative: **Staeger - Sperling**
**Müllerstrasse 3**
**80469 München (DE)**

(54) **DIAPER**

(57)     The present invention relates to a diaper worn by an infant. The diaper comprises a main body, for being fitted onto a body of an infant, and a plurality of illustration displaying parts, provided on the main body, for displaying illustrations that differ from each other, wherein the abovementioned illustrations are mutually relevant or integrated as a whole in terms of shape, pattern, color, concept, or combination thereof. With the present invention, a diaper exchanger can show different illustrations in order to an infant to arouse interest of the infant definitely in the changes of the illustrations. The labor of the diaper exchanger in the diaper exchanging process can thus be alleviated.

Fig. 1

## Description

Technical Field

This invention relates to a diaper to be worn by an infant.

Background Art

Conventionally, infants wear diapers since they cannot control their excretion at their own will. However in some cases, an infant dislikes the exchange of a diaper and struggles, and thus the exchange of a diaper has been burdensome, both physically and mentally, for a mother or other diaper exchanger.

Thus in order to alleviate such a burden, a diaper, provided with an illustration of a cute character, etc. that is considered to make infants happy, has been proposed (see for example, International Patent Publication No. 01/49230 Pamphlet).

With the present invention, a diaper is provided with an illustration and the light transmittance of a nonwoven fabric that forms the diaper is made 80% or more so as not to lower the impact of the illustration. An infant can thereby be pleased and the infant's will for diaper exchange can be increased.

However, the abovementioned diaper cannot arouse interest of an infant and, actually infants who can perform movements of the hands and feet, such as crawling, grab-walking, etc., dislike diaper exchange and escape or struggle, and thus the burden of diaper exchange that is placed on an exchanger was not alleviated.

Disclosure of the Invention

The present invention has been made in view of problems such as the above and an object thereof is to provide a method for determining illustrations for a diaper which can arouse interest of an infant and alleviate the labor of a diaper exchanger in the process of diaper exchange.

In order to achieve the above obj ect, the present Applicant noted that improvement of the quality of communication between a diaper exchanger (mainly a mother) and an infant during a diaper exchange process is important for lightening of the physical and mental burdens of the exchanger in the diaper exchanging process and promotion of healthy growth of the infant.

Here it is known that in general, the visual acuity of an infant is only approximately 0.04 to 0.08 at 6 months of age, 0.2 to 0.25 at 12 months of age, and 0.5 to 0. 6 at 24 months of age and that differences of character figures can be distinguished only past 1 year of age. It has also become clear from an Applicant's and other's own study that "an infant of 6 months to 17 months shows an interest in rattles, suspended merry-go-rounds, and other toys that make sounds or move, and an infant of 18 months to 24 months show an interest in a simple story development, such as peek-a-boo."

Based on the above facts, the present Applicant analyzed the various behavior patterns of infants in a diaper exchange process. As a result of this Applicant's research, it has become clear that an infant shows more interest in drastic changes among illustrations rather than in diverse types of illustrations. The present Applicant thus found a solution concerning the diaper exchange of infants and has come to complete the present invention.

More specifically, the present invention provides the following:

(1) A diaper worn by an infant, comprising a main body, for being fitted onto a body of an infant, and a plurality of illustration displaying parts, provided on the main body, for displaying illustrations that differ from each other, wherein said illustrations are mutually relevant or integrated as a whole in terms of shape, pattern, color, concept, or combination thereof.

With the present invention, a diaper exchanger can show different illustrations to an infant in order to arouse interest of the infant definitely in the change of these illustrations. The labor of the diaper exchanger in the diaper exchanging process can thus be alleviated.

(2) The above-described diaper, wherein said concept includes a story.

With the present invention, by making an infant recognize the development of a story, the infant can be made to be interested more definitely. The labor of a diaper exchanger in the diaper exchanging process can thus be alleviated further.

(3) The above-described diaper, wherein each of said illustrations contain at least one specific character figure.

Here, a character figure refers to a person, animal, plant, vehicle or other existence with an independent personality and may either be imaginary or actually existing.

With the present invention, since a character figure that makes up the illustrations arouse interest of an infant, the labor of a diaper exchanger in the diaper exchanging process can be alleviated further.

(4) The above-described diaper, wherein said character figure has a size of 4 centimeters square or more.

With the present invention, even an infant with undeveloped visual acuity can recognize the character figure definitely. When the character figure is of a size that is no more than 4 centimeters square, an infant with undeveloped visual acuity may not be able to distinguish the character figure.

Also, it is preferable that the character figure is drawn by using one or more colors with a hue such that the absolute value of *A* or *B* of the *LAB* color space of JISZ8729 is 20 or more. By doing so, the character figure can be recognized even more definitely, even by an infant with undeveloped visual acuity.

(5) The above-described diaper, wherein said illustration displaying parts are provided at the clothes side and body side of said main body.

With the present invention, for example, a diaper exchanger can turn the diaper inside out to show different illustrations to an infant in order. By doing so, the changes in these illustrations can arouse interest of the infant definitely. The labor of the diaper exchanger in the diaper exchanging process can thus be alleviated.

(6) The above-described diaper, wherein said illustration displaying parts are provided in the front of the front side and rear side of the infant.

With the present invention, for example, a diaper exchanger can spread or turn over the diaper from a state in which the diaper is folded to show different illustrations in order to an infant. By doing so, the changes in these illustrations can arouse interest of the infant definitely. The labor of the diaper exchanger in the diaper exchanging process can thus be alleviated.

(7) The above-described diaper, wherein one of said illustration displaying parts comprising a first member and another of said illustration displaying parts comprising a second member which is different from the first member.

With the present invention, since the illustration displaying parts comprise different members, illustrations with a variety of colors can be realized at low cost.

(8) The above-described diaper, wherein one of said illustration displaying parts contain moisture permeable sheets.

With the present invention, since the water vapor inside of the diaper can be released to the outside of the diaper via the moisture permeable sheets, the inside of the diaper can be prevented from becoming musty when the diaper is worn.

(9) The above-described diaper, wherein said main body has a fixing tape, and by rounding up said diaper and fixing the end of the fixing tape to the surface of said main body, the fixing tape is made to hold said diaper in a rounded state.

With the present invention, since the diaper can be held in a rounded state by the fixing tape, the diaper can hold the excrements of an infant definitely inside and be discarded readily.

(10) The above-described diaper, wherein said main body has a first portion to which said fixing tape is attachable and a second portion on which said illustration displaying parts provided, and the first portion is different from the second portion.

With the present invention, the hiding of the illustrations displayed at the illustration displaying parts can be prevented by the fixing tape and an infant can thus be made to recognize the illustrations definitely.

(11) The above-described diaper, wherein said fixing tape has a transparent part, which is attachable on said main body so that at least a part of said illustration displaying parts of said main body is covered by the transparent part.

With the present invention, since even when an illustration displaying part is covered by the transparent part of the fixing tape, the illustration displayed at the illustration displaying part can be visually recognized from the outside, an infant can be made to recognize the illustrations definitely.

(12) The above-described diaper, wherein said fixing tape are provided in front of the side of the infant.

(13) The above-described diaper, with which the age in months of the infant is 36 months or less.

(14) The above-described diaper, with which the age in months of the infant is 12 months or more and less than 24 months.

With the present invention, since an infant can be made to recognize the illustrations, the illustrations can arouse interest of the infant definitely and the labor of a diaper exchanger in the diaper exchanging process can be alleviated.

(15) The above-described diaper, with which the age in months of the infant is 18 months or more and less than 24 months.

With the present invention, since an infant can be made happy by a simple story development, such as "peek-a-boo," the diaper can be exchanged with ease.

(16) The above-described diaper, with which the infant has a weight of 6 kilograms or more and 14 kilograms or less.

(17) The above-described diaper, wherein said main body has a attaching tape, by attaching the end of the attaching tape to the surface of said main body, said main body is changed from a flat shape to a pants shape.

(18) The above-described diaper, wherein said main body has a pants shape in advance.

(19) The above-described diaper, which is disposable in a state in which the diaper holds excrements of the infant.

Brief Description of Drawings

Fig. 1 is an overall perspective view of a diaper according to a first embodiment of the present invention.
Fig. 2A is a front view of the diaper according to the abovementioned embodiment.
Fig. 2B is a rear view of the diaper according to the abovementioned embodiment.
Fig. 3 is a front view of a diaper according to a first modification example of the present invention.
Fig. 4 is a rear view of a diaper according to a second modification example of the present invention.
Fig. 5A is a diagram showing a first illustration of a diaper according to a third modification example of the present invention.
Fig. 5B is a diagram showing a second illustration of the diaper according to the abovementioned modification example.
Fig. 6A is a diagram showing a first illustration of a diaper according to a fourth modification example of the present invention.
Fig. 6B is a diagram showing a second illustration of the diaper according to the abovementioned modification example.
Fig. 7A is a diagram showing a first illustration of a diaper according to a fifth modification example of the present invention.
Fig. 7B is a diagram showing a second illustration of the diaper according to the abovementioned modification example.
Fig. 8A is a diagram showing a first illustration of a diaper according to a sixth modification example of the present invention.
Fig. 8B is a diagram showing a second illustration of the diaper according to the abovementioned modification example.
Fig. 9A is a diagram showing a first illustration of a diaper according to a seventh modification example of the present invention.
Fig. 9B is a diagram showing a second illustration of the diaper according to the abovementioned modification example.
Fig. 10A is a diagram showing a first illustration of a diaper according to an eighth modification example of the present invention.
Fig. 10B is a diagram showing a second illustration of the diaper according to the abovementioned modification example.
Fig. 11A is a diagram showing a first illustration of a diaper according to a ninth modification example of the present invention.
Fig. 11B is a diagram showing a second illustration of the diaper according to the abovementioned modification example.
Fig. 12A is a diagram showing a first illustration of a diaper according to a tenth modification example of the present invention.
Fig. 12B is a diagram showing a second illustration of the diaper according to the abovementioned modification example.
Fig. 13A is a diagram showing a first illustration of a diaper according to an eleventh modification example of the present invention.
Fig. 13B is a diagram showing a second illustration of the diaper according to the abovementioned modification example.
Fig. 14 is an overall perspective view of a diaper according to a second embodiment of the present invention.
Fig. 15 is a diagram showing a state in which the diaper according to the abovementioned embodiment is put in a flat state.
Fig. 16A is a diagram showing a state in which the diaper according to the abovementioned embodiment put in a flat state is folded.
Fig. 16B is a diagram showing a state in which the diaper according to the abovementioned embodiment put in a flat state is folded and then spread once.
Fig. 16C is a diagram showing a state in which the diaper according to the abovementioned embodiment put in a flat state is folded and spread twice.
Fig. 17 is a diagram showing a state in which a diaper according to the third embodiment is put in a flat state.
Fig. 18A is a diagram showing a state in which the diaper according to the abovementioned embodiment put in a flat state is folded.
Fig. 18B is a diagram showing a state in which the diaper according to the abovementioned embodiment put in a flat state is folded and then spread.
Fig. 19A is a front view of a diaper according to example 1 of the present invention.
Fig. 19B is a rear view of the diaper according to the abovementioned example.
Fig. 20A is a front view of a diaper according to comparative example 1 of this invention.
Fig. 20B is a rear view of the diaper according to the abovementioned comparative example.
Fig. 21A is a front view of a diaper according to comparative example 2 of this invention.
Fig. 21B is a rear view of the diaper according to the abovementioned comparative example.

Embodiments for Carrying out the Invention

A first embodiment of the present invention shall now be described based on the drawings. In the following description of the embodiments, the same components shall be provided with the same symbols and description thereof shall be omitted or simplified.

[First Embodiment]

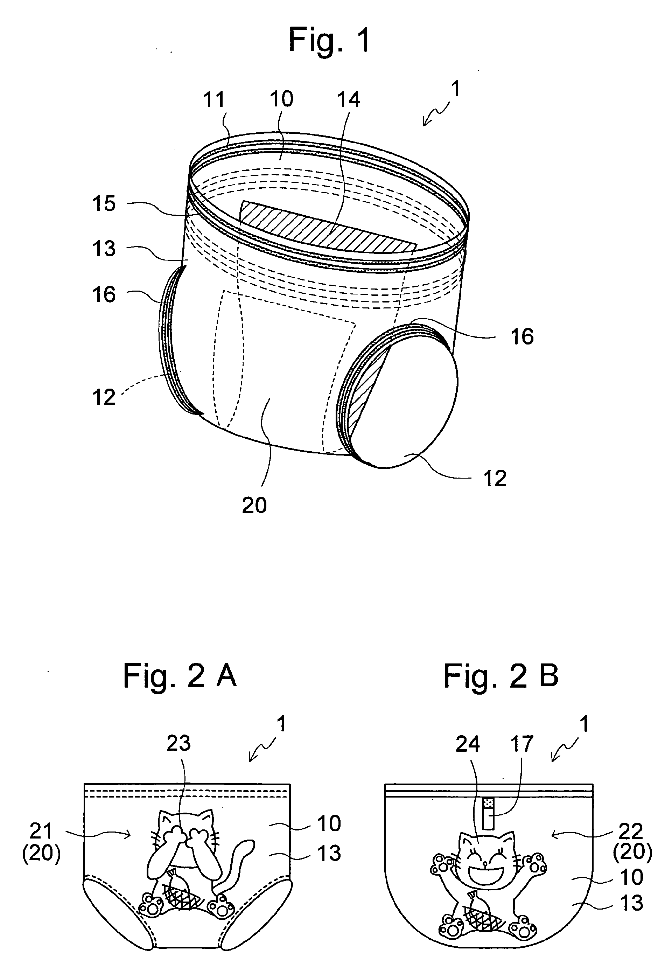

Fig. 1 shows a diaper 1 according to a first embodiment of the present invention.
Diaper 1 is worn by an infant and comprises a foldable main body 10, which is fitted onto the body of an infant, and an illustration displaying part 20, which is provided on this main body 10 and displays illustrations.
Main body 10 has the shape of pants and comprises an outer part 13, having a waist opening 11 fitted to the trunk of an infant and two leg openings 12 fitted to the legs of the infant, and an inner part 14, which extends from the front side to the rear side of waist opening 11 via the part between leg openings 12 at the inner side of outer part 13. This main body 10 can be turned over and thereby changed in position.
In order to secure resistance against fluid, good touch to skin, and air permeability, outer part 13 is arranged from a plurality of sheets. For example, hydrophobic nonwoven fabrics, non-water-permeating plastic films, or sheets formed by laminating such fabrics or films may be used as these sheets. With a plastic film, air permeability and moisture permeability can be secured by stretching after mixing in a filler.
Outer part 13 is provided with a waist tightening part 15 and leg tightening parts 16 along waist opening 11 and leg openings 12. Each of these tightening parts 15, 16 is formed by providing an elastic member between a plurality of sheets that make up outer part 13. Here, as the elastic member, a plastic sheet made of natural rubber, synthetic rubber, or thermoplastic synthetic resin may be used. This elastic member is, for example, put in a stretched state and then sandwiched between two sheets and bonded by hot melting.
Inner part 14 comprises an absorbent member provided on outer part 13 and a top sheet that covers the absorbent member. The absorbent member is formed of hydrophilic fibers and a highly absorbing polymer. As the hydrophilic fibers, absorbent fibers, such as pulp, rayon, acetate, cotton, etc., fibers prepared by hydrophilization of synthetic thermoplastic resin fibers, etc., are used. A plastic film may also be provided to retain body fluids excreted by an infant.
A hydrophilic nonwoven fabric or a liquid-permeating sheet of porous plastic, etc. is used as the top sheet. The nonwoven fabric is manufactured by a method such span lacing, needle punching, melt blowing, thermal bonding, chemical bonding, air-through method, etc. As the fibers of the nonwoven fabric, polyolefin-, polyester-, or polyamide-based fibers or sheath-core type composite fibers or side-by-side fibers formed of polyethylene, polypropylene, or ester may be used.
The above-described inner part 14 is bonded to outer part 13 using heat sealing, ultrasonic sealing, hot-melt adhesive, etc.
Illustration displaying part 20 has a first illustration displaying part 21, which, as shown in Fig. 2A, is positioned at the front surface of main body 10, and a second illustration displaying part 22, which, as shown in Fig. 2B, is positioned at the rear surface of main body 10.
First illustration display part 21 displays, as a first illustration 23, a state in which a cat (character) hides its face. Second illustration display part 22 displays, as a second illustration 24, a state in which the cat shows its face. First illustration 23 and second illustration 24 express the hiding and showing of the face by the cat, that is, a behavior of a character and are mutually related in terms of coloration.
As long as the respective illustration displaying parts 21, 22 are provided at locations that can be visually recognized from the outer side of diaper 1, the arrangement thereof is not limited in particular. For example, illustrations may be printed directly, or illustrations may be printed on films and thereafter the films may be adhered onto the nonwoven fabric or film that makes up outer part 13.
Thus in a state where an infant can visually recognize the front surface of diaper 1, the state in which the cat hides its face is shown to the infant by illustration first displaying part 21. In a state where an infant can visually recognize the rear surface of diaper 1, the state in which the cat shows its face is shown to the infant by second illustration displaying part 22.

Thus by simply turning over diaper 1, a diaper exchanger can show to the infant the behavior of a cat showing its face from a state in which it hides its face.

As shown in Fig. 2A, at the rear surface side of waist opening 11 of outer part 13 is provided a fixing tape 17 for holding diaper 1 in a rounded state. Fixing tape 17 has an adhesive part and a holding part, which is provided integrally at the tip of the adhesive part. This adhesive part is formed by coating an adhesive agent onto a plastic film.

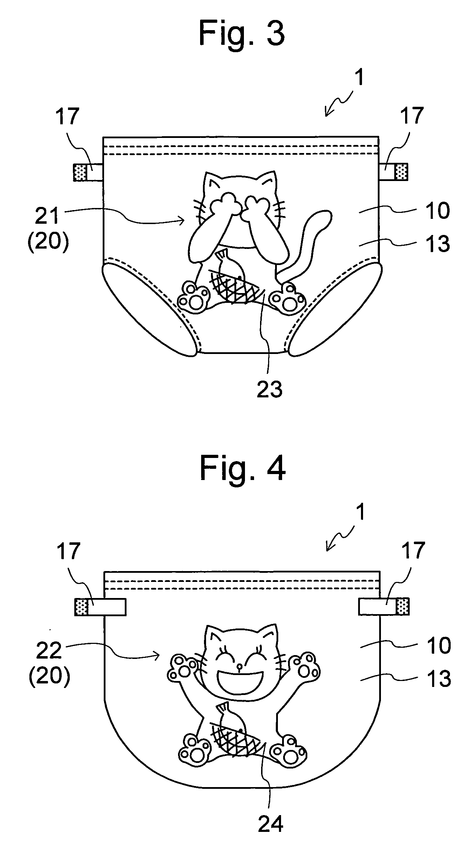

Diaper 1 is rounded up, the holding part of fixing tape 17 is held, and the adhesive part of fixing tape 17 is adhered onto the outer surface of outer part 13. Diaper 1 can thus be kept in the rounded state. This fixing tape 17 may be folded in a Z-shaped manner or may be formed from a material with stretchability. Also, the number of fixing tape 17 is not restricted in particular and a plurality thereof may be provided as shown in Figs. 3 and 4.

This fixing tape 17 is arranged so as not to overlap with first illustration 23 and second illustration 24 when the adhesive part is adhered onto the outer surface of outer part 13. The fixing tape may be made transparent so that it does not hide first illustration 23 and second illustration 24 even if it overlaps with these illustrations 23 and 24.

Here, the character (cat) displayed in illustrations 23 and 24 has a size of 8cm × 8cm. Also, one or more colors, with a hue such that the absolute value of A or B of the LAB color space of JISZ8729 is 20 or more, are used. This is because for an infant of an age in months that is targeted for use of the diaper (less than 36 months), the visual acuity is approximately 0. 04 to 0. 08 at 6 months, 0.2 to 0.25 at 12 months, and 0.5 to 0.6 at 24 months, and thus if an illustration is smaller than 8cm × 8cm or the hue is low, such an infant will not recognize the illustration even if a diaper exchanger shows diaper 1 to the infant.

Though with the present embodiment, a change in the behavior of a cat (character) is expressed by first illustration displaying part 21 and second illustration displaying part 22, the present invention is not limited thereto.

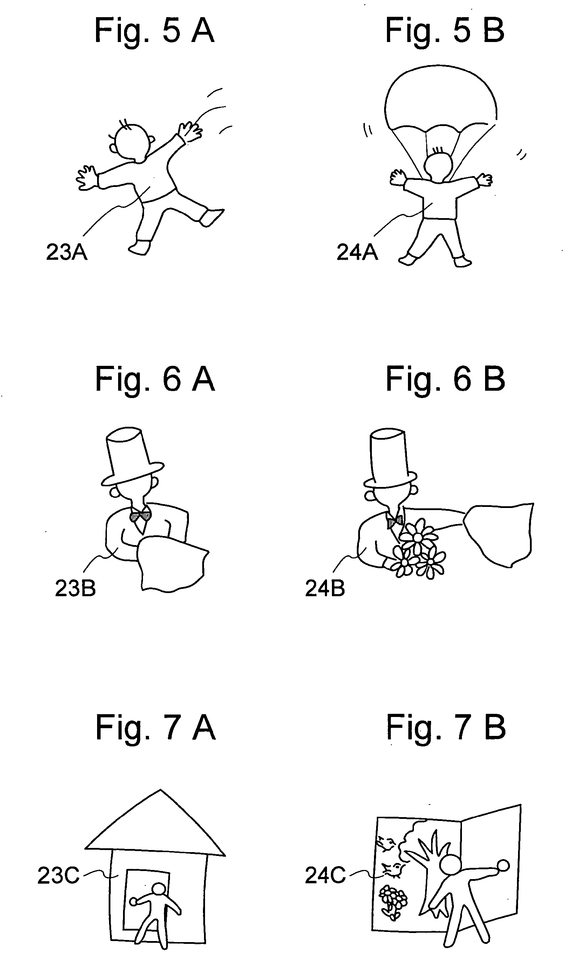

For example, a behavior and an irreversible transition in time may be expressed. To be specific, a first illustration 23A may be of a person who jumps out into air (Fig. 5A) and a second illustration 24A may be of the person with a parachute on the shoulder opening up and descending to the ground by parachute (Fig. 5B).

Or a change near the hands of a character may be expressed. To be specific, a first illustration 23B may be of a person (character) who hides his/her hands with a white cloth (Fig. 6A) and a second illustration 24B may be of the person who removes the white cloth and holds a bunch of flowers in his/her hands (Fig. 6B).

A change of scene may also be expressed. To be specific, a first illustration 23C may be that of a scene in which a person stands in front of the door of a house (Fig. 7A) and a second illustration 24C may be of a scene where a forest comes to view as the person opens the door (Fig. 7B).

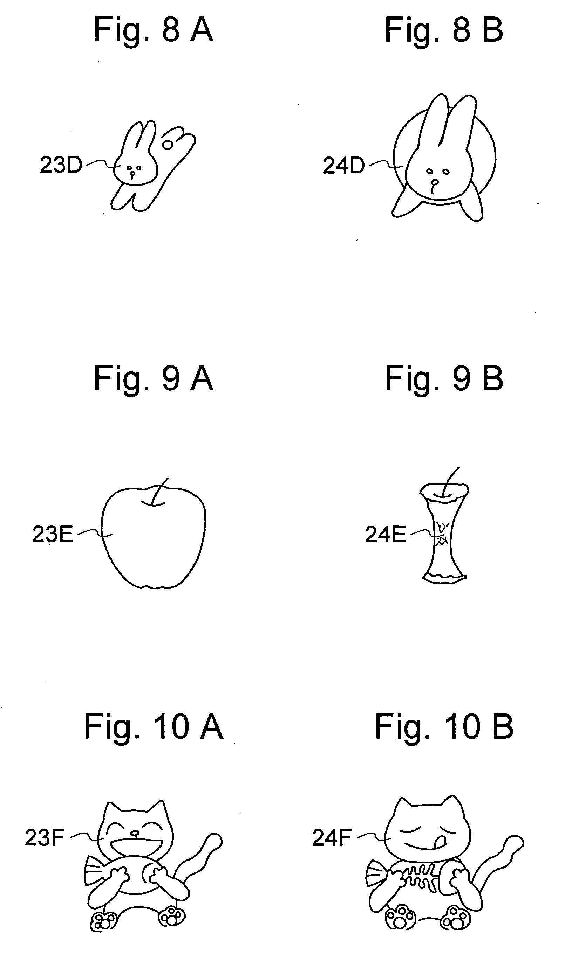

Also, a movement of a character may be expressed. To be specific, a first illustration 23D may be that of a running rabbit (character) (Fig. 8A) and a second illustration 24D may be that of the rabbit facing this way upon running through a hole in a wall (Fig. 8B).

An irreversible transition in time may also be expressed. To be specific, a first illustration 23E may be that of an apple (Fig. 9A) and a second illustration may be that of just the core of the apple after it is eaten (Fig. 9B).

A change of expression and an irreversible transition in time may also be expressed. To be specific, a first illustration 23F may be that of a cat (character) that is happily holding a fish in its hands (Fig. 10A) and a second illustration 24F may be that of the cat who is satisfied after eating the fish and is holding the bones of the fish in its hands (Fig. 10B).

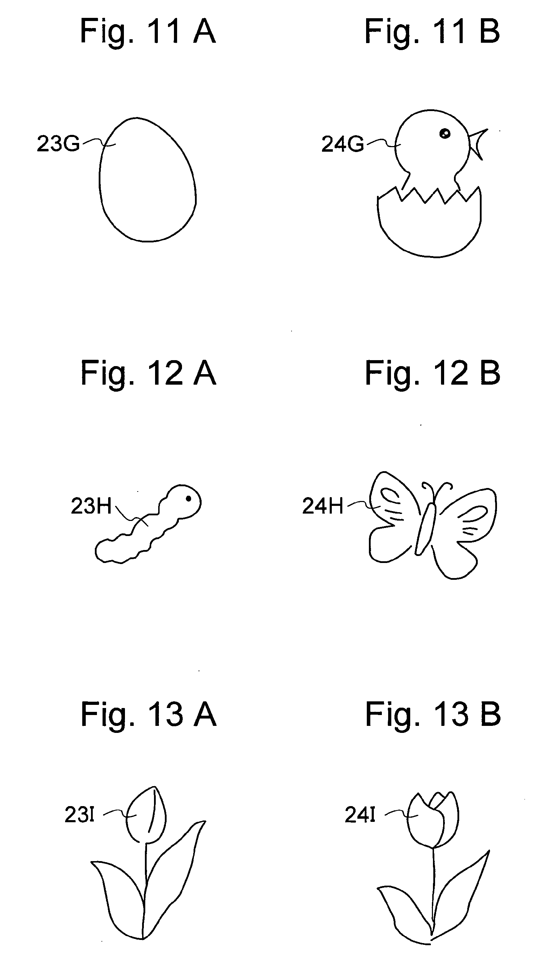

Also, a growth process may be expressed. To be specific, a first illustration 23G may be that of a chicken egg (Fig. 11A) and a second illustration 24G may be that of a chick that shows its face from the egg upon cracking of the egg (Fig. 11B). Or a first illustration 23H may be that of a larva (Fig. 12A) and second illustration 24H may be that of a butterfly (Fig. 12B). Also, a first illustration 23I may be that of a tulip in the bud state (Fig. 13A) and a second illustration 24I may be that of the tulip in bloom (Fig. 13B).

[Second Embodiment]

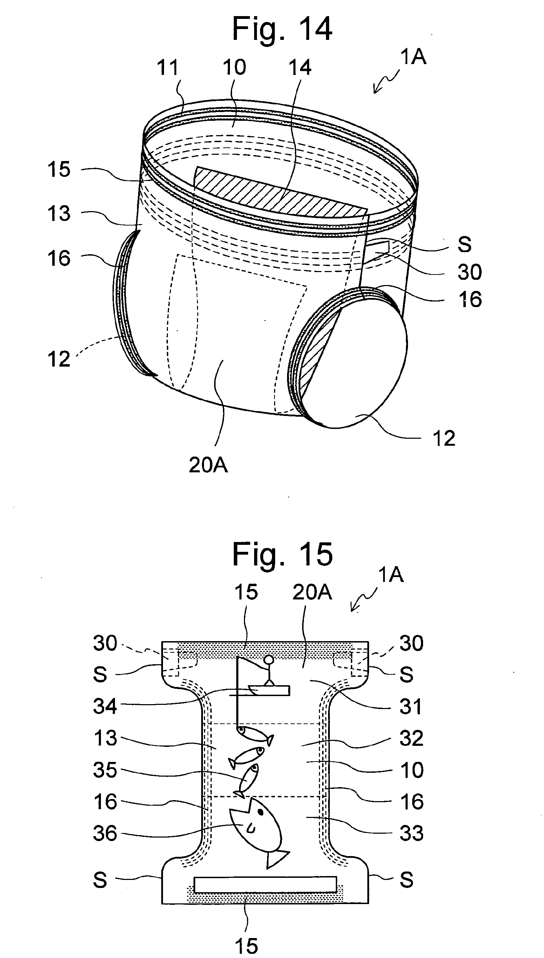

Fig. 14 is a perspective view of a diaper 1A according to a second embodiment of the present invention.

Diaper 1A is divided by cutting lines S that which extend between leg openings 12 and waist opening 11. Diaper 1 has attaching tapes 30 provided along cutting lines S.

This diaper 1A is changed from a flat shape to a pants shape by fixing the end of the attaching tapes to the surface of main body 10. Attaching tapes 30 also serve the same function as fixing tape 17 described above.

Illustration displaying part 20A has a first illustration displaying part 31, positioned at the front side of a main body 10, a second illustration displaying part 32, positioned at a groin part of main body 10, and a third illustration displaying part 33, positioned at the rear side of main body 10.

Fig. 15 shows diaper 1A in a flat state.

First illustration displaying part 31 displays a state of a person holding a fishing rod on a ship as a first illustration 34. Second illustration displaying part 32 displays a state of small fish gathering around a fishing line dropped into the sea from the fishing rod as a second illustration 35. Third illustration displaying part 33 displays a state of a large fish about to eat the small fish that gather around the fishing line as a third illustration 36.

First illustration 34, second illustration 35, and third illustration 36 thus express a single story and are mutually relevant.

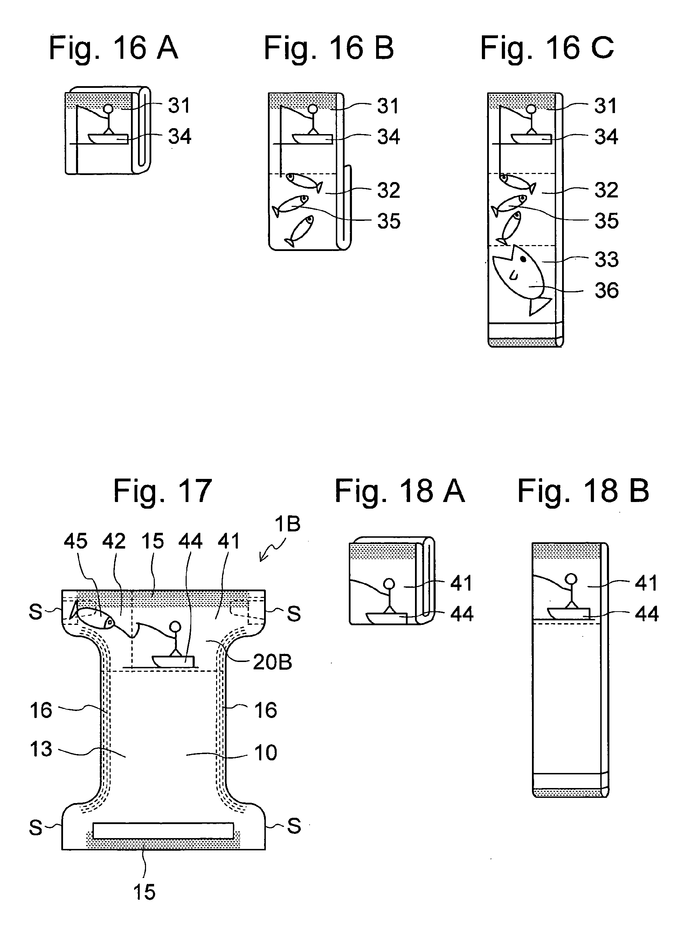

The above-described diaper 1A is used as follows. First, as shown in Fig. 16A, diaper 1A is folded in a flat state. In this state, first illustration 34 is shown to an infant. Then as shown in Fig. 16B, diaper 1A is spread once to show first illustration 34 and second illustration 35 to the infant. Subsequently, diaper 1A is spread again to show first illustration34, second illustration 35, and third illustration 36 to the infant. The infant can thus be made to recognize a story in which a fisherman drops a fishing line into the sea, small fish gather around the fishing line, and then the small fish are eaten by a large fish.

[Third Embodiment]

Fig. 17 is a diagram showing a state in which a diaper 1B according to a third embodiment of the present invention is put in a flat state.

This diaper 1B differs from the second embodiment in the arrangement of illustration displaying part 20B.

Illustration displaying part 20B has a first illustration displaying part 41, positioned at substantially the center of the front side of main body 10, and a second illustration displaying part 42, positioned at one end portion of the front side of main body 10.

First illustration displaying part 41 displays a state of a person holding a fishing rod on a ship as a first illustration 44. Second illustration displaying part 42 displays a state wherein a small fish bites the tip of a fishing line extending from the tip of the fishing rod as a second illustration 45.

First illustration 44 and second illustration 45 thus express a single story and are mutually relevant.

The above-described diaper 1B is used as follows. First as shown in Fig. 18A, diaper 1B is folded in a flat state. In this state, first illustration 44 is shown to an infant. Then after spreading diaper 1B once as shown in Fig. 18B, the respective end portion of the front side of diaper 1B are spread as shown in Fig. 17 to show first illustration 44 and second illustration 45 to the infant. The infant can thus be made to recognize a story in which a fisherman drops a fishing line into the sea and then a small fish is fished.

A diaper according to the present invention provides the following effects.

By a diaper exchanger showing different illustrations in order to an infant, interest of the infant can be arousede definitely in the changes of these illustrations. The labor of the diaper exchanger in the diaper exchanging process can thus be alleviated.

[Examples and Comparative Examples]

<Explanation of interaction value>

Communications between a diaper exchanger and an infant were evaluated using diapers of the above-described embodiments. Here, in order to evaluate communications in a quantitative manner, an index called an interaction value was used.

The interaction value is expressed by the following equation.

$$(\text{Interaction value}) = (\text{Exchanger approach value}) + (\text{Infant response value})$$

[Eq. 1]

$$(\text{Exchanger approach value}) = \sum_{\text{Start of diaper exchange}}^{\text{End of diaper exchange}} ((\text{Exchanger signal value}) \times (\text{Duration (seconds)}))$$

End of diaper exchange Σ Start of diaper exchange

[Eq. 2]

(Infant response value)

$$= \sum_{Start\ of\ diaper\ exchange}^{End\ of\ diaper\ exchange} ((Infant\ signal\ value) \times (Duration\ (seconds)))$$

(Positive action value) = Positive element of (Exchanger

approach value) + Positive element of (Infant response value)

Here, the "exchanger signal value" refers to all approaches, including crying, laughing, emitting a sound, etc., made by the exchanger to transmit the exchanger's own thought to the infant.

This "exchanger signal value" is deemed to be positive when it accompanies a positive emotion, such as joy, pleasure, etc. , and is deemed to be negative when it accompanies a negative emotion, such as discomfort, pain, etc.

The "infant signal value" refers to all approaches, including crying, laughing, emitting a sound, etc., made by the infant to transmit the infant's own thought to the exchanger in response to an approach made by the exchanger to the infant.

This "infant signal value" is deemed to be positive when it accompanies a positive emotion, such as joy, pleasure, etc., and is deemed to be negative when it accompanies a negative emotion, such as discomfort, pain, etc. To be specific, cases where the infant signal value is positive include cases where the infant laughs, emits a sound cheerfully, holds an object, moves towards the exchanger on his/her own, etc. On the other hand, cases where the infant signal value is negative include cases where the infant cries, escapes, becomes angry, struggles, etc.

The period from the "start of diaper exchange" to the "end of diaper exchange" refers to the period from the preparation of a new diaper to the guiding of the infant, removing of the diaper that the infant is already wearing, the washing of the infant's groin and putting of the new diaper, and disposal of the removed diaper.

A higher exchanger approach value signifies that the exchanger approached the child more and a higher infant response value signifies that the infant responded more to the exchanger's approach. A higher positive action value signifies that good communication was carried out more often between the exchanger and the infant. An interaction value of higher positive value signifies that in total, good communication was carried out between the exchanger and the infant.

For example, in a case where a situation in which an infant, who cries and escapes even when guided by the mother, is held down by a mother lasts for 30 seconds, a situation in which the infant laughs while being exchanged to a new diaper and the mother talks to the infant lasts for 20 seconds, and a situation in which the infant says "dada" cheerfully while the mother is throwing the diaper away lasts for 10 seconds, the above values will be as follows:

(Exchanger approach value) = (-1) $\times$ 30 + (+1) $\times$ 20 = -10

(Infant response value) = (-1) $\times$ 30 + (+1) $\times$ 20 + (+1)

$\times$ 10 = 0

(Positive action value) = 20 + 30 = 50

(Interaction value) = (-2) $\times$ 30 + (+2) $\times$ 20 + (+1) $\times$ 10

= -10

<Examination of examples and comparative examples>

The examples and comparative examples were examined using the above-described "interaction value."

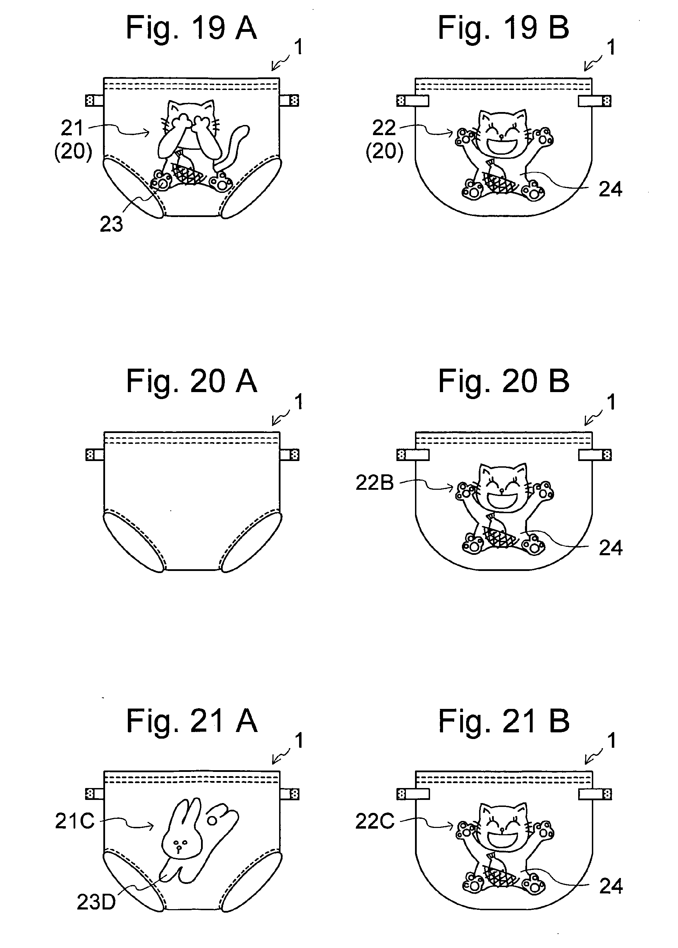

As shown in Figs. 19A and 19B, with Example 1, a first illustration displaying part 21 and a second illustration displaying part 22 are provided and a behavior of the same character is expressed by a first illustration 23 and a second illustration 24.

With Comparative Example 1, only a second illustration displaying part 22B is provided as shown in Figs. 20A and 20B.

As shown in Figs. 21A and 21B, with Comparative Example 2, a first illustration displaying part 21C and a second illustration displaying part 22C are provided and different characters are expressed by a first illustration 23D and a second illustration 24.

The above diapers were experimented with infants of the following ages in months as subjects and their mothers as diaper exchangers. The correspondence between the ages in months of the subjects and the age-in-months ranges to which the subjects belong are shown in the Table below.

**Claims**

1. A diaper worn by an infant, comprising a main body, for being fitted onto a body of an infant, and a plurality of illustration displaying parts, provided on the main body, for displaying illustrations that differ from each other, wherein said illustrations are mutually relevant or integrated as a whole in terms of shape, pattern, color, concept, or combination thereof.

2. The diaper as set forth in Claim 1, wherein said concept includes a story.

3. The diaper as set forth in Claim 1 or 2, wherein each of said illustrations contain at least one specific character figure.

4. The diaper as set forth in Claim 3, wherein said character figure has a size of 4 centimeters square or more.

5. The diaper as set forth in any one of Claim 1 to 4, wherein said illustration displaying parts are provided at the clothes side and body side of said main body.

6. The diaper as set forth in any one of Claim 1 to 4, wherein said illustration displaying parts are provided in the front of the front side and rear side of the infant.

7. The diaper as set forth in Claim 6, wherein one of said illustration displaying parts comprising a first member and another of said illustration displaying parts comprising a second member which is different from the first member.

8. The diaper as set forth in any one of Claim 1 to 7, wherein one of said illustration displaying parts contain moisture permeable sheets.

9. The diaper as set forth in any one of Claim 1 to 8, wherein said main body has a fixing tape, and by rounding up said diaper and fixing the end of the fixing tape to the surface of said main body, the fixing tape is made to hold said diaper in a rounded state.

10. The diaper as set forth in Claim 9, wherein said main body has a first portion to which said fixing tape is attachable and a second portion on which said illustration displaying parts provided, and the first portion is different from the second portion.

11. The diaper as set forth in Claim 9, wherein said fixing tape has a transparent part, which is attachable on said main body so that at least a part of said illustration displaying parts of said main body is covered by the transparent part.

12. The diaper as set forth in any one of Claim 9 to 11, wherein said fixing tape are provided in front of the side of the infant.

13. The diaper as set forth in any one of Claim 1 to 12, with which the age in months of the infant is 36 months or less.

**14.** The diaper as set forth in Claim 13, with which the age in months of the infant is 12 months or more and less than 24 months.

**15.** The diaper as set forth in Claim 14, with which the age in months of the infant is 18 months or more and less than 24 months.

**16.** The diaper as set forth in any one of Claim 1 to 15, with which the infant has a weight of 6 kilograms or more and 14 kilograms or less.

**17.** The diaper as set forth in any one of Claim 1 to 16, wherein said main body has a attaching tape, by attaching the end of the attaching tape to the surface of said main body, said main body is changed from a flat shape to a pants shape.

**18.** The diaper as set forth in any one of Claim 1 to 16, wherein said main body has a pants shape in advance.

**19.** The diaper as set forth in any one of Claim 1 to 18, which is disposable in a state in which the diaper holds excrements of the infant.

# Fig. 1

11 10 14 1

15

13

16 16

12

12

20

# Fig. 2 A

23 1

21
(20)

10

13

# Fig. 2 B

24 17 1

22
(20)

10

13

## Fig. 3

## Fig. 4

# Fig. 5 A

23A

# Fig. 5 B

24A

# Fig. 6 A

23B

# Fig. 6 B

24B

# Fig. 7 A

23C

# Fig. 7 B

24C

## Fig. 8 A

23D

## Fig. 8 B

24D

## Fig. 9 A

23E

## Fig. 9 B

24E

## Fig. 10 A

23F

## Fig. 10 B

24F

## Fig. 11 A

23G

## Fig. 11 B

24G

## Fig. 12 A

23H

## Fig. 12 B

24H

## Fig. 13 A

23I

## Fig. 13 B

24I

## Fig. 14

## Fig. 15

Fig. 16 A

Fig. 16 B

Fig. 16 C

Fig. 17

Fig. 18 A

Fig. 18 B

Fig. 19 A

1

21
(20)

23

Fig. 19 B

1

22
(20)

24

Fig. 20 A

1

Fig. 20 B

1

22B

24

Fig. 21 A

1

21C

23D

Fig. 21 B

1

22C

24

## INTERNATIONAL SEARCH REPORT

| International application No. |
| --- |
| PCT/JP03/11242 |

**A. CLASSIFICATION OF SUBJECT MATTER**
Int.Cl⁷ A61F13/514

According to International Patent Classification (IPC) or to both national classification and IPC

**B. FIELDS SEARCHED**

Minimum documentation searched (classification system followed by classification symbols)
Int.Cl⁷ A61F13/15-13/84

Documentation searched other than minimum documentation to the extent that such documents are included in the fields searched
Jitsuyo Shinan Koho          1922–1996    Jitsuyo Shinan Toroku Koho    1996–2003
Kokai Jitsuyo Shinan Koho    1971–2003    Toroku Jitsuyo Shinan Koho    1994–2003

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | WO 00/76442 A1 (Kimberly-Clark Worldwide, Inc.),<br>21 December, 2000 (21.12.00),<br>& JP 15-501211 A | 1-7,13-16,18<br>8-12,17,19 |
| Y | JP 13-54536 A (Uni-Charm Corp.),<br>27 February, 2001 (27.02.01),<br>Par. No. [0015]<br>& EP 1078620 A2 | 8 |
| Y | WO 01/21126 A1 (The Procter & Gamble Co.),<br>29 March, 2001 (29.03.01),<br>Fig. 16<br>& JP 15-509163 A | 9-12,17,19 |
| A | JP 6-106839 A (Yugen Kaisha Japan Idea),<br>19 April, 1994 (19.04.94),<br>(Family: none) | 1-19 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 14 October, 2003 (14.10.03) | 28 October, 2003 (28.10.03) |

| Name and mailing address of the ISA/<br>Japanese Patent Office | Authorized officer |
| --- | --- |
| Facsimile No. | Telephone No. |

Form PCT/ISA/210 (second sheet) (July 1998)

**INTERNATIONAL SEARCH REPORT**

| International application No. |
| --- |
| PCT/JP03/11242 |

| C (Continuation). DOCUMENTS CONSIDERED TO BE RELEVANT | | |
| --- | --- | --- |
| Category* | Citation of document, with indication, where appropriate, of the relevant passages | Relevant to claim No. |
| A | JP 12-314020 A (Wacoal Corp.), 14 November, 2000 (14.11.00), (Family: none) | 1-19 |
| A | WO 00/35401 A1 (Kimberly-Clark Worldwide, Inc.), 22 June, 2000 (22.06.00), & US 6352528 B1 | 1-19 |

Form PCT/ISA/210 (continuation of second sheet) (July 1998)